# EUROPÄISCHE PATENTANMELDUNG

(11) **EP 2 295 085 A1**
(43) Veröffentlichungstag der Anmeldung: **16.03.2011**
(21) Anmeldenummer: 10005838.7
(22) Anmeldetag: 07.06.2010
(51) Int. Cl.: A61K 49/22, A61M 5/00

(54) **Verfahren und Vorrichtung zur Herstellung eines Gasgemisches**

(30) Priorität: 17.08.2009 DE 102009037765
(71) Anmelder: Hesse, Gottfried, 80997 München (DE)
(72) Erfinder: Hesse, Gottfried, 80997 München (DE); Hoiss, Jakob, 80637 München (DE); Breu, Franz Xaver, 83700 Rottach-Egern (DE)

(57) **Zusammenfassung**

Die Erfindung bezieht sich auf ein Verfahren und eine Vorrichtung (1) zur Herstellung eines mit einem O2/CO2 Gasgemisch versetzten Medikamentes in der Anwendung zur Diagnose und Therapie des jeweiligen Zustandes bestimmter Körperteile wie auch des Blutes und seiner ggf. krankhaften Veränderungen in den Gefäßen.

## Beschreibung

Die Erfindung bezieht sich auf ein Verfahren und eine Vorrichtung zur Herstellung eines Gasgemisches zum anschließenden Injizieren des Gasgemisches mit Medikamenten in den menschlichen oder tierischen Körpers in Anwendung für Diagnose und/oder Therapie, wobei in der Diagnose mittels Ultraschall die Sichtbarmachung des jeweiligen Zustandes bestimmter Körperteile wie auch des Blutes und seiner ggf. krankhaften Veränderungen in und an den Gefäßen erzielt wird.

Nach der DE 198 12 551 C2 ist eine Vorrichtung zum Prüfen der mikrobiologischen Qualität einer definierten Menge eines druckbeaufschlagten gasförmigen Mediums bekannt. Die Vorrichtung ist definiert durch ein mit einem Sterilfilter versehenes, steriles bzw. sterilisierbares Autoklavierteil, dem das zu prüfende gasförmige Medium über ein Absperrventil druckbeaufschlagt zugeführt wird, und ein der Durchflussmessung dienendes einstellbares Messteil zum Bestimmen der zu prüfenden Gasmenge, welches Messteil über ein Dreiwegeventil mit dem Autoklavierteil verbunden ist.

Die Anwendung von Gas im menschlichen Körper ist ein Verfahren, das seit Jahrzehnten in der Medizin bekannt ist.
Bei den verschiedenen Anwendungen werden unterschiedliche, aber für die Verwendung fixe Gase verwendet oder aber auch sterile Luft. Dies betrifft auch andere Verfahren mit gasförmigen Kontrastmitteln zur Anwendung in der sonografischen Diagnostik.

Auch die Anwendung nach UK Patent Application Nos.0422307 beinhaltet eine fixe Mischung in einem Druckbehälter für einen Polidocanolschaum zur Verödung von Krampfadern. Andere Verfahren nehmen sterile Luft, die dann mit einem Arzneimittel durch eine Schaumdüse aufgeschäumt wird. Auch hier ist die Gaszusammensetzung nicht variierbar. Weiterhin hat die Anwendung von Luft den Nachteil. dass vermehrt Komplikationen auftreten können. Es ist für den Anwender nicht möglich, die Gaszusammensetzung selber individuell für den Patienten, die unterschiedlichen Gefäßgrößen, die unterschiedlichen Ziele in Therapie oder Diagnostik. oder die unterschiedlichen anzuwendenden Medikamenten festzulegen.

Eine Mischvorrichtung zur Herstellung von medizinischem Schaum bzw. zur Herstellung von Bläschen ist aus der EP 0 564 505 bekannt. Hierin ist ein Mischer mit einem wendelförmigen Mischelement beschrieben. Bei dem Mischer handelt es sich um ein Zusatzelement, das mit einer Spritze unlösbar verbunden werden kann. Beim Herausdrücken von flüssigem und/oder gasförmigem Medium aus einer zweiten Spritze gelangt das Medium in den Mischer, welcher das Gas in definiertem Volumen und Art enthält. Hierbei werden Gasphase und Flüssigphase entlang des wendelförmigen Mischelements durchmischt. Hierzu kann ein therapeutischer und/oder diagnostischer Schaum erzeugt werden. Die beschriebene Mischeinrichtung weist den Nachteil auf, dass der mit der Spritze fest verbundene Mischer, insbesondere beim Hin- und Herbewegen der Lösung, auf Grund des langen Hebels leicht abbrechen und verkantet werden kann.

Der Erfindung liegt die Aufgabe zugrunde, ein Verfahren und eine daraus resultierende Vorrichtung zu schaffen, die auf speziellen medizinischen Fachgebieten, insbesondere bei Ultra-beschallung und Röntgen von Gefäßen deutlich bessere Ergebnisse gegenüber dem Stand der Technik bzw. dem medizinischen Standard bei verminderten Risiken für Behandler und Patient zu erzielen.

Dies wird erfindungsgemäß dadurch erreicht, dass mindestens zwei verschiedene Gase als Mischung mittels Massendurchfluss- Messtechnik zur Anwendung kommen und diese je nach Anwendungsfall bzw. medizinischer Erfordernis in ihrem Mengenverhältnis zueinander entsprechend angepasst werden.
Das neue Verfahren ermöglicht in Verbindung mit der erfindungsgemäßen Vorrichtung, dass mit verschiedenen in ihrer chemischen Zusammensetzung unterschiedlich definierten Gasen eine optimale Anpassung der Mischung auch hinsichtlich ihrer in Verbindung mit unterschiedlichen Medikamenten zur Anwendung am Menschen als Individium in seiner Körper- und damit auch in seiner Gefäßstruktur, wobei eben durch die auf den jeweiligen Patienten abgestimmte Mischung eine optimale Versorgung bei minimiertem Risiko erzielt wird.

Das Verfahren ist vollständig neu und beinhaltet einen wesentlichen Fortschritt gegenüber dem Stand der Technik, da der nebenwirkungsreiche Stickstoff der Luft durch das nebenwirkungsarme CO2 ersetzt wird. Die individuelle Zusammensetzung verschiedener Gase in unterschiedlicher Konzentration ist ebenfalls bisher nicht möglich gewesen.

Nach einem weiteren Merkmal der Erfindung werden für das Aufschäumen des Gasgemisches vorzugsweise die Gase O2 und CO2 eingesetzt. Dies hat sich im Praxisversuch als die wirksamste Konstellation hinsichtlich der Abstimmung auf den jeweiligen Patienten erwiesen, wobei auch noch die verfahrenstechnischen Maßnahmen der Ansprüche 3 und 4 in den Gesamterfolg einfließen.

Zur Herstellung und Anwendung des O2/CO2- Gemisches ist eine Vorrichtung mit einem als transportablen Behälter deklarierten, mehrere Baukomponenten beinhaltende Einheit vorgesehen und an diese sind fest, aber lösbar eingangsseitig des Behälters eine diesen beaufschlagende Einheit und eine weitere Einheit ausgangsseitig des Behälters angeordnet. Solcherart ist es möglich, neben dem Hauptzweck der variablen Mischung von Gasen mittels der erfinderischen Vorrichtung auch eine ideale Lösung für Unterbringung und ggf. Transport der Vorrichtung gefunden zu haben, nämlich die problemlose Abkoppelung der vor und nach dem Behälter angebrachten Einheiten. deren ausgangsseitig befindliche Spritze von Anwendungsfall zu Anwendungsfall als steril verpackte Einheiten eingesetzt werden kann

Die Ansprüche 6 bis 10 beinhalten detaillierte Ausbildungsformen der erfinderischen Vorrichtung, wobei insbesondere der Einsatz von Massendurchfluss- Regelsystemen hervorgehoben werden muss, ermöglichen sie doch erst eine feinabgestimmte Dosierung der einzelnen Gase.

Nach einem weiteren Merkmal der Erfindung ist der Behälter aus Aluminium oder Kunststoff gefertigt und so auch als leicht transportierbarer Koffer ausgebildet, damit bei einem Einsatz außerhalb der Arztpraxis im Zusammenklang mit dem Merkmal der Abkoppelung der vor- und nachgeschalteten Aggregate ein leichter Transport wie auch Inanspruchnahme eines kleinen Stauraumes im Auto gegeben ist.

Nach einer Variante der Erfindung münden die aus der vorderen Behälterwand ins Behälterinnere verlaufenden Leitungen hinter den Massendurchfluss- Messsystemen und den nachgeschalteten Regelventilen in ein Mehrwegeventil, an dessen Ausgangsseite nur eine Leitung aus der hinteren Behälterwand hinausführt und diese dort unter Vorschaltung eines Filters mit einer einzigen Einheit, wie z.B. einer Spritze verbunden ist, wobei nach vorgegebenem Bedarf definierte Mengen von O2 und/oder CO2 über das Mehrwegeventil in eine Ausgangsleitung eingespeist werden und das Mehrwegeventil nach definierter Menge oder Zeit entweder von Hand oder über einprogrammierte Daten von der Elektronikeinheit gesteuert ist.
Auf diese Weise wird das Umstecken einer Spritze von einer O2-Leitung auf eine CO2- Leitung vermieden und stellt somit eine Arbeitserleichterung dar. Eine mögliche Verschmutzung der Spritze während des Umsteckvorganges wird so ausgeschlossen.

Die Erfindung ist in zwei Ausführungsbeispielen dargestellt:

Es zeigen:
Fig. 1 eine Vorrichtung zur Herstellung und Anwendung eines Gas-Gemisches
Fig. 2 eine Vorrichtung zur Herstellung und Anwendung eines Gas-Gemisches mit nur einer Ausgangsleitung

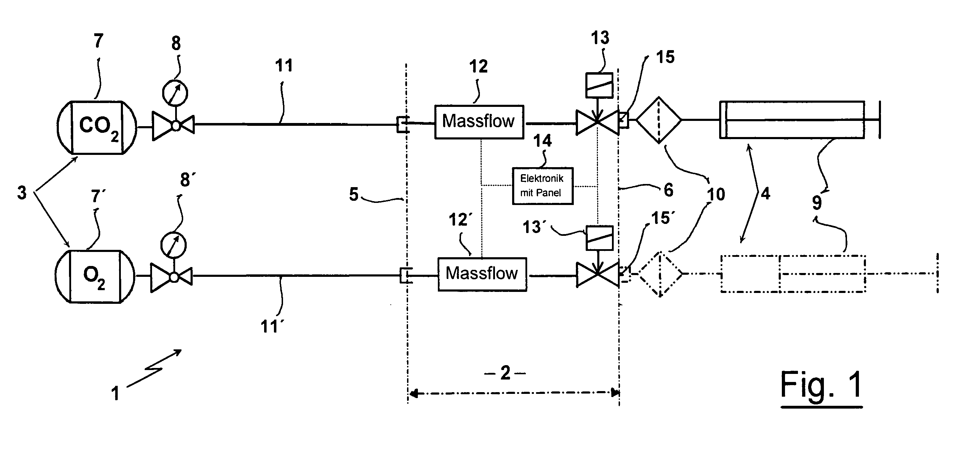

Die Fig. 1 zeigt eine Vorrichtung 1 zur Herstellung und Anwendung eines O2/CO2 Gasgemisches. Die Vorrichtung 1 setzt sich im Wesentlichen aus einem transportablen, geschlossenen Behälter 2, einer eingangsseitig 5 abkoppelbaren Einheit 3 und einer ausgangsseitig 6 ebenfalls abkoppelbaren Einheit 4 zusammen. Die Einheit 3 umfasst zwei Druckbehälter 7. 7. deren einer 7 mit CO2 gefüllt ist und der Andere 7' mit O2. Beiden vorgeschaltet sind die Reduzierventile 8, 8'. An der ausgangseitigen 6 Wand des Behälters 2 ist die Einheit 4 mit vor-geschaltetem Filtern 10 angeordnet, wobei die Einheit 4 eine Spritze 9 darstellt, die nach einer Teilbefüllung mit CO2 auf den zweiten Ausgang, dann als 9' deklariert, mit O2 restbefüllt wird. Das aus dem Druckbehälter 7 ausströmende CO2-Gas fließt über Leitung 11 in den transportablen Behälter 2, beaufschlagt hier das Massendurchfluss-Messsystem 12 mit nachgeschaltetem Regelventil 13 und fließt dann ausgangsseitig 6 des Behälters 2 über den Filter 10 in die Spritze 9. Nach Umstecken der mit CO2 teilbefüllten Spritze 9 zusammen mit dem Filter 10 auf den zweiten,.aus dem transportablen Behälter 2 führenden Strang, wird die nun als 9 deklarierte Spritze zusammen mit dem Filter 10 ihre Restbefüllung mit O2 erhalten, wobei die hier notwendigen Elemente genau denen aus dem ersten Befüllstrang entsprechen, nämlich Druckbehälter 7' für das O2- Gas mit nachgeschaltetem Reduzierventil 8' mit Manometer, Leitung 11', Massendurchfluss-Messsytem 12'. Regelventil 13', aus dem zweiten Befüllstrang über die Ausgangswand 6 des Behälters 2 austretend, in die Spritze 9 mit vorgeschaltetem Filter 10. DieMassendurchfluss- Messsysteme 12 und 12' sind auf eine Elektronikeinheit 14 geschaltet. die auch die Regelventile 13 und 13' beaufschlagt.

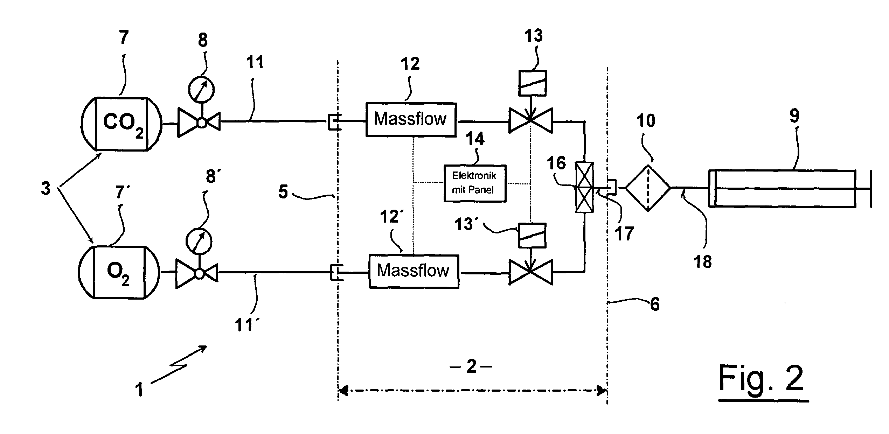

Die Fig. 2 zeigt ebenfalls eine Vorrichtung 1 zur Herstellung und Anwendung eines O2/CO2-Gasgemisches. Diese ist weitgehend identisch mit der Vorrichtung nach Fig.1, unterscheidet sich dann aber doch von ihr, indem die Leitungen 11, 11' nach den Regelventilen 13, 13' über ein Mehrwegeventil 15 in eine einzige Ausgangsleitung 18 aus dem Behälter 2 in eine Spritze 9 mit vorgeschaltetem Filter 10 enden. Solcherart entfällt das manuelle Umstecken der Spritze in der Anordnung gemäß Fig. 1. weil, über die Elektronik gesteuert, das Mehrwegeventil 16 eine vorprogrammierte Menge CO2 und eine vorprogrammierte Menge O2, dosiert zur Vollbefüllung der Spritze 9, einfließen lässt.

### - Bezugszeichenliste

- 1: Vorrichtung zur Herstellung und Anwendung eines O2/CO2-Gemisches
- 2: Transportabler Behälter
- 3: Den Behälter beaufschlagende Einheit
- 4: Den Behälter beaufschlagende Einheit
- 5: Eingangsseite von 3
- 6: Ausgangsseite von 4
- 7, 7': Druckbehälter
- 8, 8': Reduzierventil mit Manometer
- 9,: Gefäß (Spritze)
- 10,: Filter
- 11, 11': Leitungssystem
- 12, 12': Massendurchfluss- Messsystem
- 13, 13": Regelsystem
- 14: Elektronikteil mit Panel
- 15,15': Áustrittsöffnung
- 16: Mehrwegeventil
- 17: Austrittsleitung
- 18: Ausgangsleitung
- 19: .
- 20: .

## Patentansprüche

1. Verfahren zur Herstellung eines Gasgemisches zum anschließenden Injizieren des Gemisches mit Medikamenten in den menschlichen oder tierischen Körper in Anwendung für Diagnose und/oder Therapie, wobei in der Diagnose mittels Ultraschall die Sichtbarmachung des jeweiligen Zustandes bestimmter Körperteile wie auch des Blutes und seiner ggf. krankhaften Veränderungen in den Gefäßen erzielt wird, ***dadurch gekennzeichnet,* dass** mindestens zwei verschiedene Gase als Mischung mittels Massendurchfluss- Messtechniken zur Anwendung kommen und diese je nach Anwendungsfall bzw. medizinischer Erfordernis in ihrem Mengenverhältnis zueinander entsprechend angepasst werden.

2. Verfahren nach Anspruch 1, ***dadurch gekennzeichnet,* dass** für das Aufschäumen der Gasmischung vorzugsweise die Gase O2 und CO2 oder aber auch andere Gase eingesetzt werden.

3. Verfahren nach Anspruch 1 oder 2, ***dadurch gekennzeichnet,* dass** verschiedene Medikamente mit unterschiedlichen Konzentrationen von O2- und CO2- Gemische versetzt und dann aufgeschäumt werden.

4. Verfahren nach den Ansprüchen 1 bis 3, ***dadurch gekennzeichnet,* dass** Darstellung (Diagnose) und Verschluss (Therapie) krankhafter Gefäße mittels unterschiedlicher Zusammensetzung von CO2 und O2 und unterschiedlichen, frei wählbaren Medikamenten erfolgen.

5. Vorrichtung nach einem oder mehreren der Verfahrens-Ansprüche 1 bis 4, ***dadurch gekennzeichnet,* dass** zur Herstellung und Anwendung des O2/CO2- Gemisches eine Vorrichtung (1) mit einem als transportablen Behälter (2) deklarierten, mehrere Baukomponenten beinhaltende Einheit vorgesehen ist, und an diese sind fest, aber lösbar eingangsseitig (5) des Behälters (2) eine diesen beaufschlagende Einheit (3) und eine weitere Einheit (4) ausgangsseitig (6) des Behälters (2) angeordnet.

6. Vorrichtung nach Anspruch 5, ***dadurch gekennzeichnet,* dass** eingangs (5) des Behälters (2) voneinander unabhängige Druckbehälter (7, 7') angeordnet sind, deren einer (7) das CO2 Gas und deren anderer (7') das O2 Gas beinhaltet und beiden Behältern (7, 7') vor dem Durchtritt in den transportablen Behälter (2) je ein Reduzierventil (8, 8') mit Manometer nachgeschaltet ist.

7. Vorrichtung nach den Ansprüchen 5 oder 6, ***dadurch gekennzeichnet,* dass** ausgangs (6) des Behälters (2) nach Austritt aus demselben an der Behälterwand mindestens zwei voneinander unabhängige Austrittsöffnungen (15, 15') angeordnet sind, aus denen die Gase in definierter Zusammensetzung und Menge unabhängig voneinander entnommen werden können, wobei Spritze (9) nach Teilefüllung mit CO2 von Hand auf den zweiten Befüllstrang für O2 umgesteckt wird.

8. Vorrichtung nach Anspruch 7, ***dadurch gekennzeichnet,* dass** dem Gefäß /Spritze (9,) ein Filter (10) vorgeschaltet sind, dessen Porenweite vorzugsweise zwei Micron beträgt.

9. Vorrichtung nach einem oder mehreren der Ansprüche 5 bis 8, ***dadurch gekennzeichnet,* dass** im Behälter (2) zwei voneinander unabhängige Leitungssysteme (11, 11') mit je einem Massendurchfluss- Messsystem (12, 12') und diesen nachgeschalteten Regelventile (13, 13') angeordnet sind. die eingangs (5) mit den vor der Behälterwand angeordneten Druckbehälter (7, 7') und ausgangs (6) mit den nach der Behälterwand angeordneten Spritze (9) verbunden ist, wobei die Massendurchfluss- Messsysteme (12, 12') auf eine Elektronikeinheit (14) mit Panel geschaltet sind.

10. Vorrichtung nach einem oder mehreren der Ansprüche 1 bis 9, ***dadurch gekennzeichnet,* dass** der Behälter (2), vorzugsweise aus Leichtmetall oder Kunststoff gefertigt, als tragbarer Koffer ausgebildet ist.

11. Vorrichtung. insbesondere nach den Verfahrensansprüchen 1 bis 4 und dem Vorrichtungsanspruch 5 ***dadurch gekennzeichnet,* dass** die aus der vorderen Behälterwand (5) ins Behälterinnere (2) verlaufenden Leitungen (11, 11') hinter den Massendurchfluss-Messsystemen (12, 12') und den nachgeschalteten Regelventilen (13, 13') in ein Mehrwegeventil (16) münden, an dessen Ausgangsseite nur eine Austrittsleitung (17) aus der hinteren Behälterwand (6) herausführt und diese dort unter Vorschaltung eines Filters (10) mit einer einzigen Einheit, wie z. B. einer Spritze (9) verbunden ist, wobei nach vorgegebenem Bedarf definierte Mengen von O2 und/oder CO2 über das Mehrwegeventil (16) in die eine Ausgangsleitung (18) eingespeist werden.

12. Vorrichtung nach Anspruch 11, ***dadurch gekennzeichnet,* dass** das Mehrwegeventil (16) nach definierter Menge oder nach definierter Zeit entweder von Hand oder über einprogrammierte Daten von der Elektronikeinheit gesteuert (14) ist.
